# FASCICULE DE BREVET EUROPEEN

(11) **EP 2 670 766 B1**
(45) Date de publication et mention de la délivrance du brevet: **07.01.2015**
(21) Numéro de dépôt: 12708556.1
(22) Date de dépôt: 30.01.2012
(51) Int. Cl.: C07K 5/10, C07K 7/06, C07K 7/08, A61K 8/64

(54) **NOUVEAUX PEPTIDES INTERVENANT DANS LA VOIE DE SIGNALISATION SCF C-KIT ET COMPOSITIONS LES COMPRENANT**
NEUE PEPTIDE MIT MODULIERENDER WIRKUNG AUF DEM SCF C-KIT SIGNALÜBERTRAGUNGSWEG UND SIE ENTHALTENDE ZUSAMMENSETZUNGEN
NEW PEPTIDES MODULATING THE SIGNALLING PATHWAY SCF C-KIT AND COMPOSITIONS COMPRISING THEM

(30) Priorité: 01.02.2011 FR 1100299
(43) Date de publication de la demande: 11.12.2013
(73) Titulaire: ISP Investments Inc., Wilmington, DE 19805 (US)
(72) Inventeur: DAL FARRA, Claude, Kerhonkson, NY 12446 (US); DOMLOGE, Nouha, 06560 Valbonne (FR); BOTTO, Jean-Marie, 06560 Valbonne (FR)
(74) Mandataire: Macquet, Christophe
(86) Numéro de dépôt international: PCT/FR2012/000035
(87) Numéro de publication internationale: WO 2012/104500

(56) Documents cités:
- WO-A1-93/20210
- WO-A2-01/31019
- WO-A2-2010/037395
- DATABASE WPI Week 200864 Thomson Scientific, London, GB; AN 2008-K74744 XP002661121, "Stem cell factor binding inhibitor contains phosphorylated polysaccharide, 7, 8-dihydropterin group containing compound and hydroxy group containing compound as active ingredients", -& JP 2008 031094 A (KAO CORPORATION) 14 février 2008 (2008-02-14)

### SEQUENCE LISTING

<110> ISP Investments Inc
<120> NOUVEAUX PEPTIDES INTERVENANT DANS LA VOIE DE SIGNALISATION SCF/C-KIT ET COMPOSITIONS LES COMPRENANT
<130> Bv PCT 11-149
<150> FR1100299
   <151> 2011-02-01
<160> 7
<170> PatentIn version 3.5
<210> 1
   <211> 8
   <212> PRT
   <213> Artificial
<220>
   <223> Synthetic peptide
<220>
   <221> MOD_RES
   <222> (8)..(8)
   <223> AMIDATION
<400> 1
   Ser Cys Arg Asp Leu Lys Lys Thr
   1               5
<210> 2
   <211> 11
   <212> PRT
   <213> Artificial
<220>
   <223> Synthetic peptide
<400> 2
   Asn Ser Ser Lys Asp Leu Lys Lys Phe Val Ala
   1 5 10
<210> 3
   <211> 8
   <212> PRT
   <213> Artificial
<220>
   <223> Synthetic peptide
<400> 3
   Cys Lys Asp Leu Lys Lys Ser Phe
   1 5
<210> 4
   <211> 11
   <212> PRT
   <213> Artificial
<220>
   <223> Synthetic peptide
<220>
   <221> MOD_RES
   <222> (11)..(11)
   <223> AMIDATION
<400> 4
   Gln Thr Arg Asp Leu Lys Lys Ser Pro Lys Val
   1 5 10
<210> 5
   <211> 8
   <212> PRT
   <213> Artificial
<220>
   <223> Synthetic peptide
<400> 5
   Asn Lys Asp Leu Lys Lys Pro Met
   1 5
<210> 6
   <211> 6
   <212> PRT
   <213> Artificial
<220>
   <223> Synthetic peptide
<220>
   <221> MOD_RES
   <222> (6)..(6)
   <223> AMIDATION
<400> 6
   His Asp Leu Lys Lys Tyr
   1               5
<210> 7
   <211> 4
   <212> PRT
   <213> Artificial
<220>
   <223> Synthetic peptide
<220>
   <221> MOD_RES
   <222> (4)..(4)
   <223> AMIDATION
<400> 7
   Asp Leu Lys Lys
   1

## Description

La présente invention se situe dans les domaines de la cosmétique et de la pharmacie. Elle se rapporte à des composés peptidiques de formule générale (I) suivante :
R₁- (AA)ₙ- X₁- X₂- X₃ -Asp - Leu - Lys - Lys - X₄ - X₅ - (AA)ₚ - R₂ en tant que composé ayant une action sur la voie de signalisation de SCF/c-Kit, ainsi que leurs utilisations dans des compositions cosmétiques et/ou pharmaceutiques pour atténuer des défauts de pigmentation de la peau.

Chez l'humain, la couleur des cheveux et de la peau est liée à des facteurs individuels (origine ethnique, sexe, âge, etc.) et à des facteurs environnementaux (notamment les saisons de l'année, la zone d'habitation, etc.). Elle est principalement déterminée par la nature et la concentration de mélanine produite par les mélanocytes. Les mélanocytes sont de larges cellules dendritiques localisées dans la couche basale de l'épiderme. Ces cellules spécialisées vont par l'intermédiaire d'organites particuliers, les mélanosomes, synthétiser la mélanine. La synthèse de la mélanine ou mélanogénèse est un processus complexe dont les mécanismes précis ne sont pas encore élucidés et qui fait intervenir schématiquement les étapes suivantes :

Tyrosine → Dopa → Dopaquinone → Dopachrome → Mélanine.

Cette mélanine joue un rôle fondamental dans la détermination de la couleur de la peau. On parle souvent d'unités épidermiques (ou élémentaires) de mélanisation qui correspondent en fait à des ensembles fonctionnels où les mélanocytes entretiennent des contacts avec un certain nombre de kératinocytes voisins, auxquels ils transfèrent les grains pigmentaires. Le nombre d'unités varie en fonction de la région corporelle. Ces unités contiennent en moyenne 1 mélanocyte pour 36 kératinocytes (bien qu'il existe des variations). Le transfert de pigment du mélanocyte aux kératinocytes se fait en 4 phases principales :
- 1 : synthèse des mélanosomes dans le mélanocyte ;
- 2 : mélanisation des mélanosomes dans le mélanocyte ;
- 3 : transfert des mélanosomes aux kératinocytes ;
- 4 : dégradation et élimination des mélanosomes dans les kératinocytes.

Au fur et à mesure que la mélanine est synthétisée dans les mélanosomes, ceux-ci se déplacent de la région périnucléaire vers l'extrémité des dendrites des mélanocytes. Par phagocytose, l'extrémité des dendrites est capturée par les kératinocytes, les membranes dégradées et les mélanosomes redistribués dans les kératinocytes. Une fois dans les kératinocytes, les mélanosomes se répartissent selon leur taille : isolément pour les plus gros, en paquets pour les plus petits (Ortonne, *et al.* 1981). Ils sont secondairement dégradés dans des vacuoles lysosomiales (Fitzpatrick *et al.* 1979).

Le transfert des mélanosomes vers les kératinocytes, tel que décrit ci-dessus, est réalisé par l'intermédiaire de nombreux processus biologiques, enzymatiques, et n'est pas, à ce jour, encore totalement élucidé. Un des acteurs de ce processus de transfert des mélanosomes, est la protéine SCF et son récepteur c-Kit. La protéine SCF (Stem Cell Factor) est le ligand agoniste naturel du récepteur c-Kit qui est un membre de la sous-famille III de la superfamille des récepteurs tyrosine kinase (RTK). Il a été démontré dans de nombreuses publications que cette voie de signalisation SCF/c-Kit jouait un rôle clé dans plusieurs processus biologiques, et notamment dans l'hématopoïèse, dans la spermatogénèse, mais aussi dans le maintien d'une homéostasie de la peau et dans la pigmentation de celle-ci (Longley J. et al, J Invest Dermatol. 1999 ; 113 : 139-140).

Il est connu que des anomalies lors du transfert des mélanosomes vers les kératinocytes peuvent entraîner des troubles pigmentaires, qu'ils soient de nature hyperpigmentaires, ou de nature hypopigmentaires. Plus particulièrement, certaines études ont montré que la voie de signalisation SCF/c-Kit pouvait réguler à la fois la prolifération et la différenciation des mélanocytes. La protéine SCF à la surface des kératinocytes épidermaux pourrait permettre une régulation des mélanocytes adjacents, via une interaction directe avec le récepteur c-Kit situé sur lesdits mélanocytes. De plus, il a été prouvé que certains facteurs de transcription cruciaux pour la synthèse de mélanine étaient activés par la voie SCF/c-Kit (Grichnik, JM et al. J Invest Dermatol. 1998 ;111 : 233-238). Ainsi, on peut donc penser que la voie SCF/c-Kit est importante pour la fonction normale des mélanocytes, et qu'il est possible que des altérations au niveau de cette voie de signalisation soient responsables de certains troubles mélanocytaires, c'est-à-dire, pigmentaires.

JP-A-20080031094 décrit un inhibiteur de la SCF d'origine végétale.

C'est en suivant cette orientation de recherche que la Demanderesse a mise en évidence que des composés peptidiques de formule générale (I) suivante :

R₁- (AA)ₙ- X₁- X₂- X₃ -Asp - Leu - Lys - Lys - X₄ - X₅ - (AA)ₚ - R₂ (I)

étaient des agents permettant d'influer sur la pigmentation de la peau et des phanères, en assurant un transfert optimal des mélanosomes vers les kératinocytes, de manière à uniformiser le teint grâce à un effet sur la voie de signalisation de SCF/c-Kit. Les composés peptidiques selon l'invention se caractérisent par le fait qu'ils :
- protègent les structures pigmentaires de la peau vis-à-vis des agressions extérieures ;
- permettent d'atténuer les défauts de pigmentation liés à l'âge ainsi que les effets du phtotovieillissement sur la peau ; et
- permettent de traiter les irrégularités du teint et d'unifier le teint.

La présente invention a donc pour premier objet un composé peptidique de formule générale suivante (I) :

R₁- (AA)ₙ- X₁- X₂- X₃ -Asp - Leu - Lys - Lys - X₄ - X₅ - (AA)ₚ - R₂ (I)

La présente invention a pour second objet une composition cosmétique comprenant, à titre de principe actif, ledit composé peptidique de formule (I).

En outre, la présente invention a pour troisième objet l'utilisation d'une composition cosmétique comprenant ledit composé peptidique de formule (I) pour (i) protéger les structures pigmentaires de la peau vis-à-vis des agressions extérieures, (ii) atténuer les défauts de pigmentation liés à l'âge et les effets du photovieillissement sur la peau, et (iii) traiter les irrégularités du teint et unifier le teint.

Enfin, la présente invention a pour quatrième objet un procédé de traitement cosmétique de la peau ou des phanères à traiter à l'aide de la composition comprenant ledit composé peptidique de formule(I).

Le premier objet de la présente invention se rapporte à un composé peptidique de formule générale (I) :

R₁- (AA)ₙ- X₁- X₂- X₃ -Asp - Leu - Lys - Lys - X₄ - X₅ - (AA)ₚ - R₂ (I)

Dans laquelle,
X₁ représente une asparagine, une sérine, une glutamine ou aucun acide aminé,
X₂ représente une sérine, une thréonine, une cystéine ou aucun acide aminé,
X₃ représente une arginine, une lysine, une histidine ou aucun acide aminé,
X₄ représente une sérine, une tyrosine, une thréonine ou aucun acide aminé,
X₅ représente une phénylalanine, une proline, une alanine, une valine ou aucun acide aminé,
AA représente un acide aminé quelconque, et n et p sont des nombres entiers compris entre 0 et 2,
R₁ représente la fonction amine primaire de l'acide aminé N-terminal, -NH2, dans laquelle un des deux atomes d'hydrogène peut être substitué soit par une chaîne alkyle de C₁ à C₃₀ saturée ou insaturée de type acétyle, soit par un groupement aromatique de type benzoyle, tosyle ou benzyloxycarbonyle,
R₂ représente le groupe hydroxyle de la fonction carboxyle de l'acide aminé C-terminal, - OH, dans lequel l'atome d'hydrogène peut être substitué par une chaîne alkyle de C₁ à C₃₀, ou un groupement NH2, NHY ou NYY, dans lequel Y représente une chaîne alkyle de C₁ à C₄,
ladite séquence de formule générale (I) étant constituée de 4 à 13 résidus d'acides aminés.

Le terme « composé peptidique » ou « peptide » désigne un enchaînement de deux ou plusieurs acides aminés liés entre eux par des liaisons peptidiques ou par des liaisons peptidiques modifiées.

Par « composé peptidique » ou « peptide », il faut entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

Les acides aminés constituant le composé peptidique selon l'invention peuvent être sous configuration Lévogyre, c'est-à-dire L-, et/ou Dextrogyre, c'est-à-dire D-. Le peptide selon l'invention peut donc être sous forme L-, D- ou DL-.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans les domaines de la cosmétique et de la pharmacie. De préférence, on utilise pour protéger la fonction amine primaire de l'acide aminé N-terminal, une substitution par un groupement R₁ de type acyle possédant une chaîne alkyle de C₁ à C₃₀, saturée ou insaturée, pouvant être choisie parmi un groupement acétyle ou un groupement aromatique. De préférence, on utilise pour protéger la fonction carboxyle de l'acide aminé C-terminal, une substitution par un groupement R₂ de type chaîne alkyle de C₁ à C₃₀, ou un groupement NH₂, NHY ou NYY avec Y représentant une chaîne alkyle de C₁ à C₄.

Le peptide selon l'invention peut être protégé au niveau de l'extrémité N-terminale, C-terminale ou au niveau des deux extrémités.

Dans un premier mode de réalisation de l'invention, dans la formule générale (I), n et p sont égaux à zéro et la séquence de formule générale (I) est constituée de 4 à 9 résidus d'acides aminés. Ceci signifie donc que dans la formule générale (I) :
X₁ représente une asparagine, une sérine, une glutamine ou aucun acide aminé,
X₂ représente une sérine, une thréonine, une cystéine ou aucun acide aminé,
X₃ représente une arginine, une lysine, une histidine ou aucun acide aminé,
X₄ représente une sérine, une tyrosine, une thréonine ou aucun acide aminé,
X₅ représente une phénylalanine, une proline, une alanine, une valine ou aucun acide aminé,
les nombres entiers n et p sont égaux à zéro,
R₁ représente la fonction amine primaire de l'acide aminé N-terminal, -NH2, dans laquelle un des deux atomes d'hydrogène peut être substitué soit par une chaîne alkyle de C₁ à C₃₀ saturée ou insaturée de type acétyle, soit par un groupement aromatique de type benzoyle, tosyle ou benzyloxycarbonyle,
R₂ représente le groupe hydroxyle de la fonction carboxyle de l'acide aminé C-terminal, - OH, dans lequel l'atome d'hydrogène peut être substitué par une chaîne alkyle de C₁ à C₃₀, ou un groupement NH2, NHY ou NYY, dans lequel Y représente une chaîne alkyle de C₁ à C₄,
ladite séquence de formule générale (I) étant constituée de 4 à 9 résidus d'acides aminés.

Dans un second mode de réalisation préféré, le composé peptidique correspond à une des formules suivantes :
(SEQ ID n°1) Ser - Cys - Arg - Asp - Leu - Lys - Lys - Thr - NH₂
(SEQ ID n°2) Asn - Ser - Ser - Lys - Asp - Leu - Lys - Lys - Phe - Val - Ala
(SEQ ID n°3) Cys - Lys - Asp - Leu - Lys - Lys - Ser - Phe
(SEQ ID n°4) Gln - Thr - Arg - Asp - Leu - Lys - Lys - Ser - Pro - Lys - Val- NH₂
(SEQ ID n°5) Asn - Lys - Asp - Leu - Lys - Lys - Pro - Met
(SEQ ID n°6) His - Asp - Leu - Lys - Lys - Tyr - NH₂
(SEQ ID n°7) Asp - Leu - Lys - Lys - NH₂

L'invention concerne aussi des formes homologues de ces séquences. Le terme « homologue » désigne, selon l'invention, toute séquence peptidique identique à au moins 50 %, ou de préférence au moins 80 %, et encore plus préférentiellement à au moins 90 % de ladite séquence peptidique, choisie parmi les séquences SEQ ID n° 1 à SEQ ID n° 7. Par « séquence peptidique identique à au moins X % », on entend désigner un pourcentage d'identité entre les résidus d'acides aminés des deux séquences à comparer, obtenu après l'alignement optimal des deux séquences. L'alignement optimal est obtenu à l'aide d'algorithmes d'homologies locales tels que ceux utilisés par le logiciel informatique BLAST P disponible sur le site NCBI.

Le terme « homologue » peut également désigner un peptide qui diffère de la séquence d'un peptide de séquence SEQ ID n° 1 à SEQ ID n° 7 par la substitution d'acides aminés chimiquement équivalents, c'est-à-dire par la substitution d'un résidu par un autre possédant les mêmes caractéristiques. Ainsi, les substitutions classiques se font entre Ala, Val, Leu et Ile ; entre Ser et Thr ; entre les résidus acides Asp et Glu ; entre Asn et Gln ; et entre les résidus basiques Lys et Arg ; ou entre les résidus aromatiques Phe et Tyr.

Le peptide de formule générale (I) selon l'invention peut être obtenu soit par synthèse chimique classique (en phase solide ou en phase homogène liquide), soit par synthèse enzymatique (Kullmann et al., J. Biol. Chem. 1980 ; 225 : 8234) à partir d'acides aminés constitutifs ou de leurs dérivés.

Le peptide selon l'invention peut être d'origine naturelle bu synthétique. Préférentiellement selon l'invention, le peptide est obtenu par synthèse chimique.

Enfin, le principe actif peut être un peptide unique, un mélange de peptides ou de dérivés peptidiques et/ou constitué de dérivés d'acides aminés.

Le composé peptidique selon l'invention peut être utilisé à titre de médicament.

Selon un mode de réalisation avantageux de l'invention, le composé peptidique selon l'invention est solubilisé dans un ou plusieurs solvants physiologiquement adaptés, classiquement utilisés par l'homme du métier, comme l'eau, le glycérol, l'éthanol, le propanediol, le butylène glycol, le dipropylène glycol, les diglycols éthoxylés ou propoxylés, les polyols cycliques, ou tout mélange de ces solvants.

Selon encore un autre mode de réalisation avantageux de l'invention, le composé peptidique selon l'invention est solubilisé dans un vecteur cosmétique ou pharmaceutique comme les liposomes, ou adsorbé sur des polymères organiques poudreux, des supports minéraux comme les talcs et bentonites, et plus généralement solubilisé dans, ou fixé sur, tout vecteur physiologiquement adapté.

Le second objet de la présente invention se rapporte à une composition cosmétique comprenant à titre de principe actif ledit composé peptidique de formule générale (I). De manière préférée, les compositions selon l'invention se présentent sous une forme adaptée à l'application par voie topique comprenant un milieu cosmétiquement acceptable. Par « cosmétiquement acceptable », on entend des milieux qui conviennent à une utilisation en contact avec la peau ou les phanères humains, sans risque de toxicité, d'incompatibilité, d'instabilité, de réponse allergique et autres. Les compositions destinées à être appliquées sur la peau peuvent se présenter sous forme de crème, émulsion huile-dans-eau, ou eau-dans-huile ou émulsion multiple, solution, suspension, microémulsion, gel aqueux ou anhydre, sérum, ou encore de dispersion de vésicules, de patch, de spray, d'onguent, de pommade, de lotion, de colloïde, de lait, de lotion, de stick ou encore de poudre, tous adaptés à une application sur la peau, les lèvres et/ou les phanères.

Préférentiellement, ledit composé peptidique est présent dans la composition à une concentration comprise entre 0,0005 et 500 ppm environ, et préférentiellement à une concentration comprise entre 0,01 et 5 ppm.

Encore plus préférentiellement, la composition selon l'invention contient, en outre, au moins un autre principe actif. On peut citer, de manière non limitative, les classes d'ingrédients suivantes : d'autres agents actifs peptidiques, des extraits de végétaux, des agents cicatrisants, antiâge, antirides, apaisants, anti-radicalaires, anti-UV, des agents hydratants, anti-inflammatoires, anesthésiques, des agents modulant la différenciation, la pigmentation ou la dépigmentation cutanée etc.

Dans un mode plus particulier de réalisation, la composition selon l'invention comprendra, outre le composé peptidique de formule (I) :
- un (ou plusieurs) composé activateur du cytochrome c, et/ou ;
- un (ou plusieurs) composé activateur des aquaporines et/ou ;
- un (ou plusieurs) composé activateur des sirtuines et/ou ;
- un (ou plusieurs) composé qui augmente l'adhésion cellulaire et/ou ;
- un (ou plusieurs) composé qui augmente la production de protéines matricielles type collagène, laminine etc. ;
- un (ou plusieurs) composé modulateur des protéines hsp ;
- un (ou plusieurs) composé qui augmente l'énergie cellulaire ;
- un (ou plusieurs) composé modulateur de la pigmentation comme un extrait peptidique de levure, d'amaranthe, de lin, de haricot, de cacao, de maïs, de soja, de tournesol, de colza ou de pois ;
- un (ou plusieurs) composé améliorant la fonction barrière de la peau ;
- un (ou plusieurs) composé protecteur de la mitochondrie.

Lesdits composés ci-dessus peuvent être naturels, comme des hydrolysats peptidiques de végétaux, ou encore d'origine synthétique, comme des composés peptidiques.

En outre, des additifs tels que des solvants, des diluants, des colorants, des filtres solaires, des agents auto-bronzants, des pigments, des charges, des conservateurs, des absorbeurs d'odeurs, des agents épaississants, émulsifiants, humectants, émollients, des parfums, des antioxydants, des agents filmogènes, chélatants, séquestrants, conditionnants peuvent être ajoutés à la composition.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent être, par exemple, compris entre 0,01 et 20 % du poids total de la composition. Lorsque la composition de l'invention est une émulsion, la phase grasse peut représenter de 5 à 80 % en poids, et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés dans une proportion allant de 0,3 à 30 % en poids par rapport au poids total de la composition.

Un troisième objet de l'invention se rapporte à l'utilisation d'une composition cosmétique comprenant ledit composé peptidique dans un milieu cosmétiquement acceptable pour protéger les structures pigmentaires de la peau vis-à-vis des agressions extérieures. Par « structures pigmentaires » on entend les mélanocytes et les kératinocytes entretenant des contacts entre eux et formant une unité épidermique (ou élémentaire) de mélanisation (plus exactement un mélanocyte pour 36 kératinocytes en moyenne).

Par « agressions extérieures », on entend les agressions que peut produire l'environnement. A titre d'exemple, on peut citer des agressions telles que la pollution, les rayonnements UV, les agressions provoquant des stress oxydatifs, ou encore les produits à caractère irritant tels que les tensioactifs, les conservateurs ou les parfums, les agressions mécaniques, telles que les abrasions, le rasage ou l'épilation. Cependant, préférentiellement, les agressions extérieures sont principalement constituées par les rayonnements UV, et en particulier les UVB et les agressions induisant des stress oxydatifs.

Une autre utilisation du peptide selon l'invention consiste à atténuer les défauts de pigmentation liés à l'âge et les effets du photovieillissement sur la peau. Par « photovieillissement », on entend le vieillissement prématuré de la peau causé par l'exposition prolongée et cumulative au soleil. Par « défauts de pigmentation liés à l'âge », on entend les tâches séniles, les lentigos solaires, les tâches de dépigmentation, ou encore les éphélides. A cet effet, le peptide selon l'invention peut être utilisé pour traiter les irrégularités du teint et unifier le teint.

Enfin, un dernier objet de la présente invention se rapporte à un procédé de traitement cosmétique caractérisé par l'application, le matin et le soir sur la peau, d'une composition comprenant le peptide selon l'invention afin d'atténuer les défauts de pigmentation liés à l'âge et les effets du photovieillissement sur la peau. Un premier mode de réalisation consiste à l'application de ladite composition soit avant une exposition au soleil, en tant que soin avant-soleil, soit après une exposition au soleil, en tant que soin après-soleil, afin (i) de prévenir et/ou de réparer les dommages dus aux rayonnements UV sur les cellules de la peau et (ii) de limiter l'apparition de tâches d'hyperpigmentation.

Les exemples suivants décrivent et démontrent l'efficacité de composés peptidiques tels que décrits selon l'invention mais ne doivent pas être interprétés comme une limitation de la présente invention.

La figure 1 présente les résultats, sous forme d'histogrammes, d'une quantification de l'intensité de la mélanine présente dans des biopsies de peau humaine traitées ou non à l'aide d'un composé peptidique selon l'invention et d'un inhibiteur de la voie de signalisation SCF/c-Kit.

Les figures 2a et 2b sont des histogrammes présentant les résultats de 2 tests des comètes réalisés sur des kératinocytes humains normaux (NHK) et des mélanocytes épidermiques humains normaux (NHEM) soumis à des rayonnements UVB et traités ou non à l'aide d'un composé peptidique selon l'invention.

### Exemple 1 : Fontana-Masson sur des biopsies de peau humaine traitées ou non avec le composé peptidique de SEQ ID n°6 à 1 % et un inhibiteur de c-Kit

Des biopsies issues d'échantillons de peau humaine sont mises en culture à l'interface air/liquide. Les biopsies sont alors maintenues en culture pendant 48 heures, traitées ou non avec le composé peptidique SEQ ID n°6 à 1 %, à raison de trois applications par jour et traitées ou non avec un inhibiteur spécifique de l'activité c-Kit, l'imatinib, à une concentration de 20 µM à raison d'une application par jour.

Les biopsies sont ensuite fixées dans du formaldéhyde puis incluses dans de la paraffine. Des coupes de 4 µm sont alors réalisées et déposées sur des lames *Superfrost Plus* (Thermo Scientific). La coloration de la mélanine sur les coupes est réalisée à l'aide d'une solution de nitrate d'argent à 10 % chauffée à 60°C et révélée par ajout de la solution de sodium thiosulfate à 5 %. Les coupes de peau sont alors examinées au microscope dans le visible (Nikon Eclipse E600 microscope).

### Résultats :

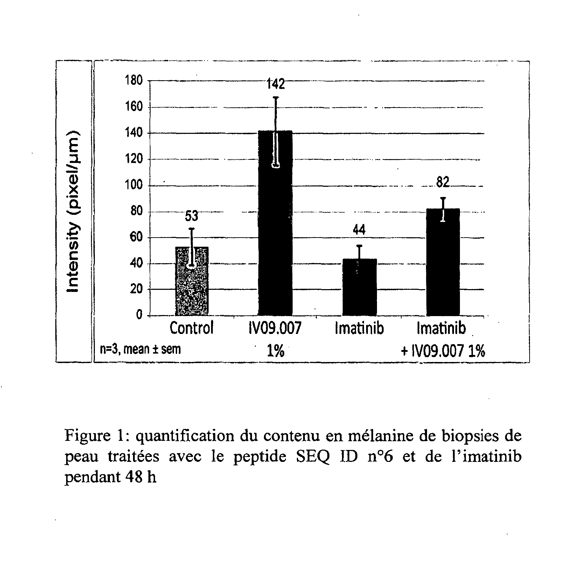

L'ensemble des résultats est présenté dans la figure 1.

L'intensité de la mélanine en pixel/µm a été mesurée dans 4 conditions : contrôle, avec l'actif, avec l'imatinib, et avec l'actif et l'imatinib. Lorsque l'actif est ajouté au milieu de culture sans inhibiteur, on observe une augmentation de l'intensité de la mélanine passant de 53 à 142 pixels/µm par rapport à la condition contrôle. On observe ainsi l'effet de l'actif sur l'intensité de la mélanine.

Lorsque l'inhibiteur de c-Kit est ajouté au milieu (sans actif), on observe une chute de l'intensité de la mélanine, passant de 53 pixels/µm en condition contrôle à 44 pixels/µm. Ceci confirme ainsi l'effet inhibiteur de l'imatinib sur la quantité de mélanine présente. Lorsque l'imatinib est ajouté dans le milieu de culture en présence de l'actif peptidique, on constate que l'effet inhibiteur de l'imatinib est renversé par l'effet activateur du composé selon l'invention, sur la voie de signalisation c-Kit. L'intensité de la mélanine passe de 44 pixels/µm en condition avec l'imatinib seul, à 82 pixels/µm en condition avec le composé peptidique et l'imatinib.

### Conclusions :

On observe qu'après 48 heures de traitement, le composé peptidique SEQ ID n°6 induit une hausse du contenu de mélanine dans la couche basale de l'épiderme et permet de renverser l'effet dépigmentant induit par l'imatinib, suggérant ainsi une action spécifique de l'actif peptidique sur la voie de signalisation impliquant c-Kit. Les effets du composé peptidique selon l'invention pourraient donc contribuer à une meilleure protection de la couche basale de l'épiderme.

### Exemple 2 : Etude par micro-array de l'expression d'une sélection de gènes dans des cellules NHK traitées ou non avec le composé peptidique SEQ ID n°3 à 1 %

Des kératinocytes humains normaux (ou NHK) sont mis en culture pendant 48 heures et sont traités avec le composé peptidique SEQ ID n°3 à une concentration de 1 %. Une condition contrôle sans ingrédient actif est aussi réalisée.

Les cellules sont ensuite détachées de leur support à l'aide de trypsine, puis centrifugées à 1500 tours/min pendant 10 minutes afin de les concentrer. Les cellules sont resuspendues dans du PBS 1X et 8 volumes de PreProtect (Miltenyi Biotec) afin de stabiliser les ARNs. Les ARNs totaux sont alors extraits et amplifiés à l'aide du kit µMACS One-step T7 Templates (Miltenyi Biotec). Les ARNs sont reverse-transcrits et marqués avec les fluorochromes Cy5 et Cy3. Les ADNc fluorescents ainsi obtenus sont hybridés sur des lames, sur lesquelles sont déposées, en quadrupliquets, des gènes exprimés dans la peau (PIQOR^{™} Skin Microarray). Ceci permet d'obtenir le profil d'expression des ces gènes dans les cellules NHK traitées ou non avec le composé peptidique selon l'invention. Les données présentées sont obtenues à partir d'un seul échantillon poolé (n=1). Un ratio supérieur à 1,48 indique une régulation positive et un ratio plus faible que 0,58 montre une régulation négative. La caractérisation de la fonction des gènes est réalisée en utilisant les logiciels en ligne DAVID Bioinformatics Resources ("Database for Annotation, Visualization and Integrated Discovery").

### Résultats :

Les résultats sont présentés dans le tableau 1 ci-dessous.

**Tableau 1 : résultats d'une expérience de micro-array sur l'expression d'une sélection de gènes sur des NHK traités avec le composé SEQ ID n°3 à 1% versus NHK non traités**

| Genes: | Ratios: | | Genes: | Ratios: | |
|---|---|---|---|---|---|
| **DNA repair and synthésis:** | | | **Extracellular matrix and cutaneous barrier:** | | |
| **OGG1** | *8-OXOGUANINE DNA GLYCOSYLASE* | 1.86 | **SPARC** | *SECRETED PROTEIN ACIDIC AND RICH IN CYSTEINE* | 0.20 |
| **MUTYH** | *A*/*G-SPECIFICADENINE DNA GLYCOSYLASE* | 2.74 | **TIMP2** | *METALLOPROTEINASE INHIBITOR 2 PRECURSOR* | 0.55 |
| **RAD17** | *CELL CYCLE CHECKPOINT PROTEIN RAD17* | 2.02 | **LAMA5** | *LAMININ ALPHA 5 CHAIN* | 2.45 |
| **RAD54L** | *DNA REPAIR AND RECOMBINATION PROTEIN RAD54-LIKE* | 2.93 | **MMP2** | *MATRIX METALLOPROTEINASE*-*2* | 1.85 |
| **XPA** | *DNA REPAIR PROTEIN COMPLEMENTING XP-A CELLS* | 1.48 | **KRT19** | *KERATIN*, *TYPE I CYTOSKELETAL* 19 | 1.57 |
| **ERCC2** | *DNA EXCISION REPAIR PROTEIN ERGC-2* | 3.85 | **GLUL** | *GLUTAMINE SYNTHETASE* | 1.78 |
| **ERCC5** | *DNA REPAIR PROTEIN COMPLEMENTING XP-G CELLS* | 1.52 | **TGM1** | *TRANSGLUTAMINASE* 1 | 1.74 |
| **DDB2** | *DNA DAMAGE BINDING PROTEIN 2* | 1.74 | | | |
| **TK1** | *THYMIDINE KINASE, CYTOSOLIC* | 1.92 | **Signal** | **transduction and protein phosphorylation:** | |
| **POLB** | *DNA POLYMERASE BETA* | 1.60 | **EGFR_3** | *EPIDERMAL GROWTH FACTOR RECEPTOR PRECURSOR* | 1.56 |
| **POLD1** | *DNA POLYMERASE DELTA CATALYTIC CHAIN* | 2.65 | **EGR1** | *EARLY GROWTH RESPONSE PROTEIN* 1 | 1.68 |
| **POLD4** | *DNA POLYMERASE DELTA SUBUNIT* 4 | 2.42 | | | |
| ***TYMS*** | *THYMIDYLATE SYNTHASE* | 1.77 | **PRKCG** | *PROTEIN KINASE C, GAMMA TYPE* | 1.84 |
| | | | **PRKDC** | *DNA-DEPENDENT PROTEIN KINASE CATALYTIC SUBUNIT* | 2.57 |
| | | | **PTK6** | *PROTEIN TYROSINE KINASE* 6 | 1.74 |
| **Immunologicals functions:** | | | **MAP2K2** | *MAP KINASE KINASE 2* | 1.62 |
| **IL128** | *INTERLEUKIN-12 BETA CHAIN PRECURSOR* | 0.16 | | | |
| **IL13** | *INTERLEUKIN-13 PRECURSOR* | 0.20 | **Cell death control:** | | |
| **IL16** | *INTERLEKIN-16 PRECURSOR* | 0.21 | **BCL10** | *B CELL LYMPHOMA*/*LEUKEMIA 10* | 0.28 |
| **IL17A** | *INTERLEUKIN-17 PRECURSOR* | 0.07 | **BCL2L2** | *BCL-2-LIKE 2 PROTEIN* | 3.09 |
| **IL1A** | *INTERLEUKIN-1 ALPHA PRECURSOR* | 0.52 | **DAD1** | *DEFENDER AGAINST CELL DEATH1* | 1.85 |
| **IL3** | *INTERLEUKIN-3 PRECURSOR* | 0.08 | | | |
| **IL5** | *INTERLEUKIN-5 PRECURSOR* | 0.12 | **Oxidative stress:** | | |
| **IL9** | *INTERLEUKIN-9 PRECURSOR* | 0.28 | **CAT** | *CATALASE* | 1.80 |

### Conclusions :

On observe que certains gènes impliqués dans le renforcement de la barrière cutanée sont modulés. On observe également une régulation vers le haut des gènes impliqués dans la transduction des signaux. Enfin, l'expression du gène de la catalase est augmentée, suggérant ainsi un effet protecteur contre les stress oxydatifs.

### Exemple 3 : Immunomarquage de l'expression de la catalase dans des biopsies de peau humaine traitées ou non avec le composé peptidique SEQ ID n°7

Afin de confirmer les résultats obtenus lors du test micro-array sur le gène de la catalase, un immunomarquage de celle-ci a été réalisé en présence ou non d'un peptide selon l'invention.

Pour cela, des biopsies issues d'échantillons de peau humaine sont mises en culture à l'interface air/liquide. Les biopsies sont alors maintenues en culture et traitées ou non avec le composé peptidique SEQ ID n°7 à des concentrations de 1 et 3 %. Ces biopsies sont ensuite fixées dans du formaldéhyde puis incluses dans de la paraffine... Des coupes de 4 µm sont réalisées et déposées sur des lames polylysine (Thermo Scientific). L'immunomarquage est alors entrepris à l'aide d'un anticorps primaire polyclonal de lapin, et spécifique de la catalase (Calbiochem) puis d'un anticorps secondaire anti-lapin couplé à un fluorochrome (Invitrogen). Les coupes de peau sont alors examinées au microscope à Epi-fluorescence (Nikon Eclipse E600 microscope).

### Résultats / conclusions:

On observe que le peptide SEQ ID n°7 selon l'invention augmente non seulement l'expression du gène de la catalase, mais aussi l'expression de la protéine correspondante dans la peau, protéine qui est essentielle dans la protection des stress oxydatifs. Par conséquent, on peut conclure que le peptide SEQ ID n°7 a un effet sur la protection des cellules de la peau contre les stress oxydatifs.

### Exemple 4 : Test des comètes sur des cellules NHK et NHEM traitées avec le composé peptidique SEQ ID n°7 à 1 % et irradiées avec des rayonnements UVB

Afin de constater l'effet protecteur des peptides selon l'invention sur des cellules NHK et NHEM et de confirmer les résultats obtenus par micro-array, un test des comètes est réalisé en soumettant lesdites cellules à des rayonnements UVB.

Pour cela, des NHK et NHEM sont mis en culture pendant 24 heures en présence du composé peptidique SEQ ID n°7 à une concentration de 1 %, puis irradiées avec des rayonnements UVB à raison de 30 mJ/cm² pour les NHEM et 60 mJ/cm² pour les NHK. Une condition contrôle est également réalisée sans actif peptidique. Les cellules sont ensuite détachées de leur support à l'aide de trypsine, puis centrifugées à 900 tours/min pendant 5 minutes afin de les concentrer et de les dénombrer.

Un nombre défini de cellules (25 000 cellules) est ensuite inclut dans un gel d'agarose Low Melting à 0.75 %, puis déposé sur une lame de verre préalablement recouverte d'agarose à 1 %. Les lames sont ensuite immergées dans une solution de lyse pendant 1 h 30 à 4 °C, puis dans une solution alcaline pendant 20 minutes à 4 °C. Les cellules sont ainsi lysées et l'ADN dénaturé. Les lames sont plongées dans une solution d'électrophorèse avant d'appliquer un champ électrique (20 V - 250 mA). L'ADN ainsi dénaturé est soumis à une migration au sein du gel d'agarose à 4 °C. L'application d'un colorant fluorescent de l'ADN sur les lames (l'iodure de propidium à 2 µg/ml) permet d'observer au microscope l'ADN apparaissant sous forme de comètes dans le cas où il a été endommagé.

Enfin, un logiciel de quantification permet de déterminer le *Tail Moment* moyen appliqué à chaque condition testée. Ce paramètre renseigne sur le niveau d'endommagement de l'ADN : plus il est élevé, plus les dommages sur l'ADN sont importants.

### Résultats :

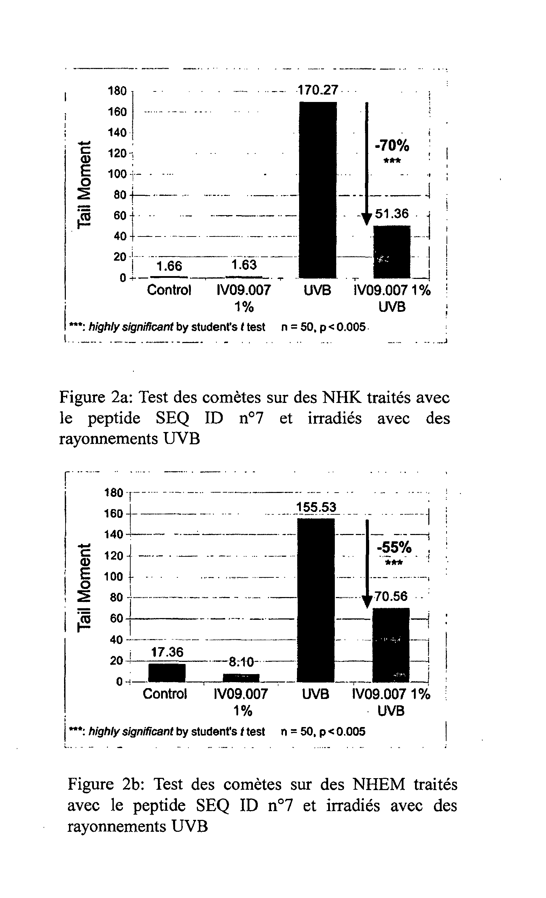

Les résultats sont présentés dans les figures 2a et 2b. La figure 2a présente le Tail Moment d'un test des comètes réalisé sur des cellules NHK. La figure 2b présente le Tail Moment d'un test des comètes réalisé sur des cellules NHEM.

Pour les NHK, on observe qu'en présence d'UVB, le Tail Moment est très élevé (170.27 contre 1.66 en contrôle). Ceci témoigne des effets délétères des rayonnements UVB sur l'ADN des cellules. On observe un résultat équivalent dans les cellules NHEM. Par contre, lorsque le peptide SEQ ID n°7 est appliqué en prétraitement sur les cellules, avant traitement par des rayonnements UVB, on observe une baisse du Tail Moment dans les NHK, passant de 170.27 en condition UVB à 51.36 en condition actif + UVB, soit une baisse de 70 %. Une baisse de 55 % est constatée dans les cellules NHEM.

### Conclusions :

Les résultats de ces tests des comètes confirment ceux obtenus lors du test micro-array. Le composé peptidique SEQ ID n°7 a permis d'avoir une action protectrice sur l'ADN des cellules et a, de manière générale, une action protectrice contre les stress oxydatifs.

### Exemple 5 : Composition d'une crème solaire

| Noms commerciaux | Noms INCI | % massique |
|---|---|---|
| PHASE A | | |
| Eau déminéralisée | Aqua (Water) | qsp |
| Pemulen TR1 | Acrylates/C10-30 Alkyl | 0,40 |
| | Acrylate Crosspolymer | |
| Glycerine | Glycerin | 3,00 |
| Nipagin M | Sodium Methylparaben | 0,3 |

| PHASE B | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Phenoxetol | Phenoxyethanol | 0,5 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Peptide SEQ ID n°4 | | 3 ppm |
| Parfum | Parfum (Fragrance) | qsp |
| Colorant | | qsp |

## Revendications

1. Composé peptidique de formule générale suivante (I) :
R₁- (AA)ₙ- X₁- X₂- X₃ -Asp - Leu - Lys - Lys - X₄ - X₅ - (AA)ₚ - R₂
dans laquelle,
X₁ représente une asparagine, une sérine, une glutamine ou aucun acide aminé,
X₂ représente une sérine, une thréonine, une cystéine ou aucun acide aminé,
X₃ représente une arginine, une lysine, une histidine ou aucun acide aminé,
X₄ représente une sérine, une tyrosine, une thréonine ou aucun acide aminé,
X₅ représente une phénylalanine, une proline, une alanine, une valine ou aucun acide aminé,
AA représente un acide aminé quelconque, et n et p sont des nombres entiers compris entre 0 et 2,
R₁ représente la fonction amine primaire de l'acide aminé N-terminal, -NH2, dans laquelle un des deux atomes d'hydrogène peut être substitué soit par une chaîne alkyle de C₁ à C₃₀ saturée ou insaturée de type acétyle, soit par un groupement aromatique de type benzoyle, tosyle ou benzyloxycarbonyle,
R₂ représente le groupe hydroxyle de la fonction carboxyle de l'acide aminé C-terminal, - OH, dans lequel l'atome d'hydrogène peut être substitué par une chaîne alkyle de C₁ à C₃₀, ou un groupement NH2, NHY ou NYY, dans lequel Y représente une chaîne alkyle de C₁ à C₄,
ladite séquence de formule générale (I) étant constituée de 4 à 13 résidus d'acides aminés, **caractérisé en ce qu'**il correspond à une des formules suivantes :
(SEQ ID n°1) Ser - Cys - Arg - Asp - Leu - Lys - Lys - Thr - NH₂
(SEQ ID n°2) Asn - Ser - Ser - Lys - Asp - Leu - Lys - Lys - Phe - Val - Ala - NH₂
(SEQ ID n°3) Cys - Lys - Asp - Leu - Lys - Lys - Ser - Phe
(SEQ ID n°4) Gln - Thr - Arg - Asp - Leu - Lys - Lys - Ser - Pro - Lys - Val- NH₂
(SEQ ID n°5) Asn - Lys - Asp - Leu - Lys - Lys - Pro - Met
(SEQ ID n°6) His - Asp - Leu - Lys - Lys - Tyr - NH₂
(SEQ ID n°7) Asp - Leu - Lys - Lys - NH₂

2. Composé peptidique selon la revendication 1, **caractérisé en ce qu'**il est solubilisé dans un ou plusieurs solvants physiologiquement adaptés, comme l'eau, le glycérol, l'éthanol, le propanediol, le butylène glycol, le dipropylène glycol, les diglycols éthoxylés ou propoxylés, les polyols cycliques, ou tout mélange de ces solvants.

3. Composition cosmétique comprenant à titre de principe actif ledit composé peptidique selon l'une des revendications 1 ou 2.

4. Composition cosmétique selon la revendication 3, **caractérisée en ce qu'**elle se présente sous une forme adaptée à l'application par voie topique comprenant un milieu cosmétiquement acceptable.

5. Composition selon l'une des revendications 3 ou 4, **caractérisée en ce que** ledit composé peptidique est présent dans la composition à une concentration comprise entre 0,0005 et 500 ppm environ, et préférentiellement à une concentration comprise entre 0,01 et 5 ppm.

6. Composition selon l'une quelconque des revendications 3 à 5, **caractérisée en ce qu'**elle continent, en outre, au moins un autre principe actif.

7. Composition selon la revendication 6, **caractérisée en ce que** ledit principe actif est choisi parmi des hydrolysats peptidiques issus de végétaux, d'autres composés peptidiques, des filtres solaires, des agents anti-radicalaires, ou encore des agents antirides.

8. Utilisation cosmétique d'une composition comprenant un composé peptidique de formule générale suivante (I) :
R₁- (AA)ₙ- X₁- X₂- X₃ -Asp - Leu - Lys - Lys - X₄ - X₅ - (AA)ₚ - R₂
dans laquelle,
X₁ représente une asparagine, une sérine, une glutamine ou aucun acide aminé,
X₂ représente une sérine, une thréonine, une cystéine ou aucun acide aminé,
X₃ représente une arginine, une lysine, une histidine ou aucun acide aminé,
X₄ représente une sérine, une tyrosine, une thréonine ou aucun acide aminé,
X₅ représente une phénylalanine, une proline, une alanine, une valine ou aucun acide aminé,
AA représente un acide aminé quelconque, et n et p sont des nombres entiers compris entre 0 et 2,
R₁ représente la fonction aminé primaire de l'acide aminé N-terminal, -NH2, dans laquelle un des deux atomes d'hydrogène peut être substitué soit par une chaîne alkyle de C₁ à C₃₀ saturée ou insaturée de type acétyle, soit par un groupement aromatique de type benzoyle, tosyle ou benzyloxycarbonyle,
R₂ représente le groupe hydroxyle de la fonction carboxyle de l'acide aminé C-terminal, - OH, dans lequel l'atome d'hydrogène peut être substitué par une chaîne alkyle de C₁ à C₃₀, ou un groupement NH2, NHY ou NYY, dans lequel Y représente une chaîne alkyle de C₁ à C₄,
ladite séquence de formule générale (I) étant constituée de 4 à 13 résidus d'acides aminés et un milieu cosmétiquement acceptable pour protéger les structures pigmentaires de la peau vis-à-vis des agressions extérieures.

9. Utilisation selon la revendication 8 **caractérisé en ce que** les agressions extérieures sont les rayonnements UV.

10. Utilisation selon la revendication 8 pour atténuer les défauts de pigmentation liés à l'âge et les effets du photovieillissement sur la peau.

11. Utilisation selon la revendication précédente **caractérisée en ce que** les défauts de pigmentation liés à l'âge sont les tâches séniles, les lentigos solaires, les tâches de dépigmentation, ou encore les éphélides.

12. Utilisation selon la revendication 8 pour traiter les irrégularités du teint et unifier le teint.

13. Procédé de traitement cosmétique **caractérisé en ce que** l'on applique, matin et soir sur la peau, une composition comprenant une quantité efficace d'un composé peptidique de formule générale suivante (I) :
R₁- (AA)ₙ- X₁-X₂- X₃ -Asp - Leu - Lys - Lys - X₄ - X₅ - (AA)ₚ - R₂
dans laquelle,
X₁ représente une asparagine, une sérine, une glutamine ou aucun acide aminé,
X₂ représente une sérine, une thréonine, une cystéine ou aucun acide aminé,
X₃ représente une arginine, une lysine, une histidine ou aucun acide aminé,
X₄ représente une sérine, une tyrosine, une thréonine ou aucun acide aminé,
X₅ représente une phénylalanine, une proline, une alanine, une valine ou aucun acide aminé,
AA représente un acide aminé quelconque, et n et p sont des nombres entiers compris entre 0 et 2,
R₁ représente la fonction amine primaire de l'acide aminé N-terminal, -NH2, dans laquelle un des deux atomes d'hydrogène peut être substitué soit par une chaîne alkyle de C₁ à C₃₀ saturée ou insaturée de type acétyle, soit par un groupement aromatique de type benzoyle, tosyle ou benzyloxycarbonyle,
R₂ représente le groupe hydroxyle de la fonction carboxyle de l'acide aminé C-terminal, - OH, dans lequel l'atome d'hydrogène peut être substitué par une chaîne alkyle de C₁ à C₃₀, ou un groupement NH2, NHY ou NYY, dans lequel Y représente une chaîne alkyle de C₁ à C₄,
ladite séquence de formule générale (I) étant constituée de 4 à 13 résidus d'acides aminés, pour atténuer les défauts de pigmentation liés à l'âge et les effets du photovieillissement sur la peau.

14. Procédé de traitement cosmétique selon la revendication précédente **caractérisé en ce que** la composition est appliquée avant une exposition au soleil, en tant que soin avant-soleil, ou après une exposition au soleil en tant que soin après-soleil, afin de prévenir et/ou réparer les dommages dus aux rayonnements UV sur les cellules de la peau.

15. Procédé de traitement cosmétique selon la revendication 13 **caractérisé en ce que** la composition est appliquée avant une exposition au soleil, en tant que soin avant-soleil, ou après une exposition au soleil, en tant que soin après-soleil, afin de limiter l'apparition de tâches d'hyperpigmentation.

## Patentansprüche

1. Peptidverbindung mit der folgenden allgemeinen Formel (I) :
R₁-(AA)ₙ-X₁-X₂-X₃-Asp-Leu-Lys-Lys-X₄-X₅-(AA)ₚ-R₂,
in der
X₁ für ein Asparagin, ein Serin, ein Glutamin oder keine Aminosäure steht,
X₂ für ein Serin, ein Threonin, ein Cystein oder keine Aminosäure steht,
X₃ für ein Arginin, ein Lysin, ein Histidin oder keine Aminosäure steht,
X₄ für ein Serin, ein Tyrosin, ein Threonin oder keine Aminosäure steht,
X₅ für ein Phenylalanin, ein Prolin, ein Alanin, ein Valin oder keine Aminosäure steht,
AA für eine beliebige Aminosäure steht und n und p ganze Zahlen zwischen 0 und 2 sind,
R₁ für die primäre Aminofunktion der N-terminalen Aminosäure, -NH₂, steht, in der eines von zwei Wasserstoffatomen entweder durch eine gesättigte oder ungesättigte C₁- bis C₃₀-Alkylkette vom Acetyl-Typ oder durch eine aromatische Gruppe vom Benzoyl-, Tosyl- oder Benzyloxycarbonyl-Typ substituiert sein kann,
R₂ für die Hydroxylgruppe der Carboxylfunktion der C-terminalen Aminosäure, -OH, steht, in der das Wasserstoffatom durch eine C₁- bis C₃₀-Alkylkette oder eine NH₂-, NHY- oder NYY-Gruppe substituiert sein kann, in der Y für eine C₁- bis C₄-Alkylkette steht,
wobei die besagte Sequenz der allgemeinen Formel (I) aus 4 bis 13 Aminosäureresten besteht, **dadurch gekennzeichnet, dass** sie einer der folgenden Formeln entspricht:
(SEQ ID Nr. 1) Ser-Cys-Arg-Asp-Leu-Lys-Lys-Thr-NH₂
(SEQ ID Nr. 2) Asn-Ser-Ser-Lys-Asp-Leu-Lys-Lys-Phe-Val-Ala-NH₂
(SEQ ID Nr. 3) Cys-Lys-Asp-Leu-Lys-Lys-Ser-Phe
(SEQ ID Nr. 4) Gln-Thr-Arg-Asp-Leu-Lys-Lys-Ser-Pro-Lys-Val-NH₂
(SEQ ID Nr. 5) Asn-Lys-Asp-Leu-Lys-Lys-Pro-Met
(SEQ ID Nr. 6) His-Asp-Leu-Lys-Lys-Tyr-NH₂
(SEQ ID Nr. 7) Asp-Leu-Lys-Lys-NH₂.

2. Peptidverbindung nach Anspruch 1, **dadurch gekennzeichnet, dass** sie in einem oder mehreren physiologisch angepassten Lösungsmitteln solubilisiert wird, wie Wasser, Glycerin, Ethanol, Propandiol, Butylenglykol, Dipropylenglykol, ethoxylierte oder propoxylierte Diglykole, cyclische Polyole oder jegliches Gemisch dieser Lösungsmittel.

3. Kosmetische Zusammensetzung, die die besagte Peptidverbindung nach einem der Ansprüche 1 oder 2 als Wirkstoff umfasst.

4. Kosmetische Zusammensetzung nach Anspruch 3, **dadurch gekennzeichnet, dass** sie in einer zur topischen Anwendung angepassten Form vorliegt, die ein kosmetisch akzeptables Medium umfasst.

5. Zusammensetzung nach einem der Ansprüche 3 oder 4, **dadurch gekennzeichnet, dass** die besagte Peptidverbindung in der Zusammensetzung in einer Konzentration zwischen ungefähr 0,0005 und 500 ppm und vorzugsweise in einer Konzentration zwischen 0,01 und 5 ppm vorliegt.

6. Zusammensetzung nach einem der Ansprüche 3 bis 5, **dadurch gekennzeichnet, dass** sie außerdem mindestens einen anderen Wirkstoff enthält.

7. Zusammensetzung nach Anspruch 6, **dadurch gekennzeichnet, dass** der besagte Wirkstoff aus Peptidhydrolysaten, die von Pflanzen stammen, anderen Peptidverbindungen, Sonnenschutzmitteln, Radikalfängern oder auch Antifaltenmitteln ausgewählt ist.

8. Kosmetische Verwendung einer Zusammensetzung, die eine Peptidverbindung mit der folgenden allgemeinen Formel (I) umfasst:
R₁-(AA)ₙ-X₁-X₂-X₃-Asp-Leu-Lys-Lys-X₄-X₅-(AA)ₚ-R₂,
in der
X₁ für ein Asparagin, ein Serin, ein Glutamin oder keine Aminosäure steht,
X₂ für ein Serin, ein Threonin, ein Cystein oder keine Aminosäure steht,
X₃ für ein Arginin, ein Lysin, ein Histidin oder keine Aminosäure steht,
X₄ für ein Serin, ein Tyrosin, ein Threonin oder keine Aminosäure steht,
X₅ für ein Phenylalanin, ein Prolin, ein Alanin, ein Valin oder keine Aminosäure steht,
AA für eine beliebige Aminosäure steht und n und p ganze Zahlen zwischen 0 und 2 sind,
R₁ für die primäre Aminofunktion der N-terminalen Aminosäure, -NH₂, steht, in der eines von zwei Wasserstoffatomen entweder durch eine gesättigte oder ungesättigte C₁- bis C₃₀-Alkylkette vom Acetyl-Typ oder durch eine aromatische Gruppe vom Benzoyl-, Tosyl- oder Benzyloxycarbonyl-Typ substituiert sein kann,
R₂ für die Hydroxylgruppe der Carboxylfunktion der C-terminalen Aminosäure, -OH, steht, in der das Wasserstoffatom durch eine C₁- bis C₃₀-Alkylkette oder eine NH₂-, NHY- oder NYY-Gruppe substituiert sein kann, in der Y für eine C₁- bis C₄-Alkylkette steht,
wobei die besagte Sequenz der allgemeinen Formel (I) aus 4 bis 13 Aminosäureresten besteht, in einem kosmetisch akzeptablen Medium zum Schützen der Pigmentstrukturen der Haut vor äußeren Einflüssen.

9. Verwendung nach Anspruch 8, **dadurch gekennzeichnet, dass** die äußeren Einflüsse UV-Strahlen sind.

10. Verwendung nach Anspruch 8 zum Mildern von altersbedingten Pigmentstörungen und Auswirkungen der Lichtalterung auf die Haut.

11. Verwendung nach dem vorhergehenden Anspruch, **dadurch gekennzeichnet, dass** die altersbedingten Pigmentstörungen Altersflecken, Lentigos solaris, Pigmentflecken oder auch Sommersprossen sind.

12. Verwendung nach Anspruch 8 zum Behandeln von Unregelmäßigkeiten des Teints und Egalisieren des Teints.

13. Verfahren zur kosmetischen Behandlung, **dadurch gekennzeichnet, dass** am Morgen und am Abend eine Zusammensetzung auf die Haut aufgebracht wird, wobei die Zusammensetzung eine wirksame Menge einer Peptidverbindung mit der folgenden allgemeinen Formel (I) umfasst:
R₁-(AA)ₙ-X₁-X₂-X₃-Asp-Leu-Lys-Lys-X₄-X₅-(AA)ₚ-R₂,
in der
X₁ für ein Asparagin, ein Serin, ein Glutamin oder keine Aminosäure steht,
X₂ für ein Serin, ein Threonin, ein Cystein oder keine Aminosäure steht,
X₃ für ein Arginin, ein Lysin, ein Histidin oder keine Aminosäure steht,
X₄ für ein Serin, ein Tyrosin, ein Threonin oder keine Aminosäure steht,
X₅ für ein Phenylalanin, ein Prolin, ein Alanin, ein Valin oder keine Aminosäure steht,
AA für eine beliebige Aminosäure steht und n und p ganze Zahlen zwischen 0 und 2 sind,
R₁ für die primäre Aminofunktion der N-terminalen Aminosäure, -NH₂, steht, in der eines von zwei Wasserstoffatomen entweder durch eine gesättigte oder ungesättigte C₁- bis C₃₀-Alkylkette vom Acetyl-Typ oder durch eine aromatische Gruppe vom Benzoyl-, Tosyl- oder Benzyloxycarbonyl-Typ substituiert sein kann,
R₂ für die Hydroxylgruppe der Carboxylfunktion der C-terminalen Aminosäure, -OH, steht, in der das Wasserstoffatom durch eine C₁- bis C₃₀-Alkylkette oder eine NH₂-, NHY- oder NYY-Gruppe substituiert sein kann, in der Y für eine C₁- bis C₄-Alkylkette steht,
wobei die besagte Sequenz der allgemeinen Formel (I) aus 4 bis 13 Aminosäureresten besteht, zum Mildern von altersbedingten Pigmentstörungen und Auswirkungen der Lichtalterung auf die Haut.

14. Verfahren zur kosmetischen Behandlung nach dem vorhergehenden Anspruch, **dadurch gekennzeichnet, dass** die Zusammensetzung vor einer Sonnenaussetzung als ein Sonnenschutzmittel oder nach einer Sonnenaussetzung als ein After-Sun-Mittel aufgebracht wird, um Schäden der Zellen der Haut aufgrund von UV-Strahlen zu verhindern und/oder zu reparieren.

15. Verfahren zur kosmetischen Behandlung nach Anspruch 13, **dadurch gekennzeichnet, dass** die Zusammensetzung vor einer Sonnenaussetzung als ein Sonnenschutzmittel oder nach einer Sonnenaussetzung als ein After-Sun-Mittel aufgebracht wird, um das Auftreten hyperpigmentierter Flecken zu begrenzen.

## Claims

1. Peptide compound having the following general formula (I):
R₁-(AA)ₙ-X₁-X₂-X₃-Asp-Leu-Lys-Lys-X₄-X₅-(AA)ₚ-R₂
wherein,
X₁ represents an asparagine, a serine, a glutamine or no amino acid,
X₂ represents a serine, a threonine, a cysteine or no amino acid,
X₃ represents an arginine, a lysine, a histidine or no amino acid,
X₄ represents a serine, a tyrosine, a threonine, or no amino acid,
X₅ represents a phenylalanine, a proline, an alanine, a valine or no amino acid,
AA represents any amino acid, and n and p are integers between 0 and 2,
R₁ represents the primary amine function of the N-terminal amino acid, -NH2, wherein one of the two hydrogen atoms may be substituted either by an acetyl type saturated or unsaturated C₁ to C₃₀ alkyl chain, or by a benzoyl, tosyl or benzyloxycarbonyl type aromatic group, R₂ represents the hydroxyl group of the carboxyl function of the C-terminal amino acid, -OH, wherein the hydrogen atom may be substituted by a C₁ to C₃₀ alkyl chain, or an NH₂, NHY or NYY group, wherein Y represents a C₁ to C₄ alkyl chain,
said sequence having general formula (I) consisting of 4 to 13 amino acid residues, **characterised in that** it corresponds to one of the following formulas:
(SEQ ID No. 1) Ser-Cys-Arg-Asp-Leu-Lys-Lys-Thr-NH₂
(SEQ ID No. 2) Asn-Ser-Ser-Lys-Asp-Leu-Lys-Lys-Phe-Val-Ala-NH₂
(SEQ ID No. 3) Cys-Lys-Asp-Leu-Lys-Lys-Ser-Phe
(SEQ ID No. 4) Gln-Thr-Arg-Asp-Leu-Lys-Lys-Ser-Pro-Lys-Val-NH₂
(SEQ ID No. 5) Asn-Lys-Asp-Leu-Lys-Lys-Pro-Met
(SEQ ID No. 6) His-Asp-Leu-Lys-Lys-Tyr-NH₂
(SEQ ID No. 7) Asp-Leu-Lys-Lys-NH₂

2. Peptide compound according to claim 1, **characterised in that** it is solubilised in one or a plurality of physiologically suitable solvents, such as water, glycerol, ethanol, propanediol, butylene glycol, dipropylene glycol, ethoxylated or propoxylated diglycols, cyclic polyols, or any mixture of these solvents.

3. Cosmetic composition comprising as an active ingredient said peptide compound according to any of claims 1 or 2.

4. Cosmetic composition according to claim 3, **characterised in that** it is presented in a form suitable for topical application comprising a cosmetically acceptable medium.

5. Composition according to any of claims 3 or 4, **characterised in that** said peptide compound is present in the composition at a concentration between approximately 0.0005 and 500 ppm, and preferentially at a concentration between 0.01 and 5 ppm.

6. Composition according to any of claims 3 to 5, **characterised in that** it further contains at least one further active ingredient.

7. Composition according to claim 6, **characterised in that** said active ingredient is chosen from plant-based peptide hydrolysates, further peptide compounds, sun filters, anti-radical agents, or anti-wrinkle agents.

8. Cosmetic use of a composition comprises a peptide compound having the following general formula (I):
R₁-(AA)ₙ-X₁-X₂-X₃-Asp-Leu-Lys-Lys-X₄-X₅-(AA)ₚ-R₂
wherein,
X₁ represents an asparagine, a serine, a glutamine or no amino acid,
X₂ represents a serine, a threonine, a cysteine or no amino acid,
X₃ represents an arginine, a lysine, a histidine or no amino acid,
X₄ represents a serine, a tyrosine, a threonine, or no amino acid,
X₅ represents a phenylalanine, a proline, an alanine, a valine or no amino acid,
AA represents any amino acid, and n and p are integers between 0 and 2,
R₁ represents the primary amine function of the N-terminal amino acid, -NH2, wherein one of the two hydrogen atoms may be substituted either by an acetyl type saturated or unsaturated C₁ to C₃₀ alkyl chain, or by a benzoyl, tosyl or benzyloxycarbonyl type aromatic group,
R₂ represents the hydroxyl group of the carboxyl function of the C-terminal amino acid, -OH, wherein the hydrogen atom may be substituted by a C₁ to C₃₀ alkyl chain, or an NH₂, NHY or NYY group, wherein Y represents a C₁ to C₄ alkyl chain,
said sequence having general formula (I) consisting of 4 to 13 amino acid residues and a cosmetically acceptable medium for protecting the pigment structures of the skin against external attacks.

9. Use according to claim 8 **characterised in that** the external attacks are UV rays.

10. Use according to claim 8 for attenuating age-related pigmentation defects and the effects of photoageing on skin.

11. Use according to the above claim **characterised in that** the age-related pigmentation defects are age spots, liver spots, depigmentation spots, or ephiledes.

12. Use according to claim 8 for treating complexion irregularities and evening out the complexion.

13. Cosmetic treatment method **characterised in that** it consists of applying, each morning and evening onto the skin, a composition comprising an effective quantity of a peptide compound having the following general formula (I):
R₁-(AA)ₙ-X₁-X₂-X₃-Asp-Leu-Lys-Lys-X₄-X₅-(AA)ₚ-R₂
wherein,
X₁ represents an asparagine, a serine, a glutamine or no amino acid,
X₂ represents a serine, a threonine, a cysteine or no amino acid,
X₃ represents an arginine, a lysine, a histidine or no amino acid,
X₄ represents a serine, a tyrosine, a threonine, or no amino acid,
X₅ represents a phenylalanine, a proline, an alanine, a valine or no amino acid,
AA represents any amino acid, and n and p are integers between 0 and 2,
R₁ represents the primary amine function of the N-terminal amino acid, -NH2, wherein one of the two hydrogen atoms may be substituted either by an acetyl type saturated or unsaturated C₁ to C₃₀ alkyl chain, or by a benzoyl, tosyl or benzyloxycarbonyl type aromatic group, R₂ represents the hydroxyl group of the carboxyl function of the C-terminal amino acid, -OH, wherein the hydrogen atom may be substituted by a C₁ to C₃₀ alkyl chain, or an NH₂, NHY or NYY group, wherein Y represents a C₁ to C₄ alkyl chain,
said sequence having general formula (I) consisting of 4 to 13 amino acid residues, for attenuating age-related pigmentation defects and the effects of photoageing on skin.

14. Cosmetic treatment method according to the above claim **characterised in that** the composition is applied before exposure to the sun, as a pre-sun treatment, or after exposure to the sun as an after-sun treatment, in order to prevent and/or repair damage caused by UV rays on skin cells.

15. Cosmetic treatment method according to claim 13 **characterised in that** the composition is applied before exposure to the sun, as a pre-sun treatment, or after exposure to the sun as an after-sun treatment, in order to limit the appearance of hyperpigmentation spots.
